# EUROPÄISCHE PATENTANMELDUNG

(11) **EP 3 202 758 A1**
(43) Veröffentlichungstag der Anmeldung: **09.08.2017**
(21) Anmeldenummer: 16154092.7
(22) Anmeldetag: 03.02.2016
(51) Int. Cl.: C07C 209/26, C07C 227/18, C07D 207/16, C07D 295/023, C07D 211/60, C07C 211/48, C07C 211/27, C07C 229/12, C07C 229/36, C07C 229/24

(54) **REDUKTIVE ALKYLIERUNG VON AMINEN MIT ORTHOCARBONSÄUREESTERN**

(71) Anmelder: Evonik Degussa GmbH, 45128 Essen (DE)
(72) Erfinder: KADYROV, Renat, 60389 Frankturt (DE)

(57) **Zusammenfassung**

Die vorliegende Erfindung betrifft ein Verfahren zur N-Alkylierung von Aminen durch Umsetzung eines Amins mit einem Orthocarbonsäureester und mit Wasserstoff in Gegenwart eines Hydrierkatalysators.

## Beschreibung

Die vorliegende Erfindung betrifft ein Verfahren zur Herstellung von Aminen durch reduktive Alkylierung von Aminen bzw. von Ammoniak mittels Orthocarbonsäureestern in Gegenwart von Wasserstoff und von einem Hydrierkatalysator.

Amine spielen eine dominierende Rolle in zahlreichen komplexen Naturstoffen wie beispielsweise den Alkaloiden, Vitaminen oder Aminosäuren, deren chemische, pharmazeutische und industrielle Bedeutung unbestritten ist. Als Zwischenprodukte finden Amine u.a. Anwendung in der Synthese von Pharmazeutika, Agrochemikalien, Nahrungsmittelzusatzstoffen, Farbstoffen oder Kosmetika. Für den Bereich der Wirkstoffe spielen dabei Aminosäuren eine überragende Rolle.

Amine lassen sich u.a. durch Reduktion der korrespondierenden Carbonsäureamide, beispielweise durch katalytische Hydrierung von Carbonsäureamiden (M. Stein und B. Breit, Angew. Chem. Int. Ed. 2013, 52, 2231-2234; WO 2015/067450 A1) oder durch katalytische Hydrierung von Amidacetalen, Keten-N,O-Acetalen oder Esterimiden (WO 2015/067448 A1) herstellen.

Eine weitere Methode, die reduktive Alkylierung von Ammoniak bzw. von primären oder sekundären Aminen mittels Aldehyden und Ketonen, führt in Gegenwart von Wasserstoff und heterogenen Metallkatalysatoren zu einer Alkylierung der Aminogruppe. Die reduktive Alkylierung von Aminen gehört mit zu den wichtigsten Methoden für die Herstellung von Aminen (W. S. Emerson, The preparation of amines by reductive alkylation, in Organic Reactions, Vol. 4, 1948, pp. 174-255, Wiley, N.Y.; Catalytic Hydrogenation over Platinum Metals, Academic Press, New York, 1967, S. 291 ff; Catalytic Hydrogenation in Organic Synthesis, Academic Press, New York, 1979, 165 ff; F. Müller und R. Schröter in Methoden Org. Chem. (Houben-Weyl), 1957, XI/1, S. 602-671; S. Nishimura, Handbook of Heterogeneous Catalytic Hydrogenation for Organic Synthesis 2001, pp. 406-411, Wiley, N.Y.). Die Reaktion von Aldehyden oder Ketonen mit Ammoniak bzw. primären oder sekundären Aminen in Gegenwart von Wasserstoff und von heterogenen Katalysatoren erfordert in der Regel aber in Bezug auf Druck und Temperatur drastische Reaktionsbedingungen. Riermeier et al. (WO 01/05741 A1) schlagen demgegenüber eine Variante dieser Reaktion unter Verwendung von homogenen Metallkatalysatoren vor, bei welcher mildere Reaktionsbedingungen eingehalten werden können.

Die reduktive Alkylierung von Aminen in Gegenwart von Wasserstoff unter heterogener Katalyse hat jedoch weitere Nachteile. Bei der Synthese von sekundären Aminen durch Umsetzung von primären Aminen mit hochreaktiven Aldehyden gelingt es in der Regel nicht, die Reaktion auf der Stufe der Monoalkylierung zu stoppen. Dies führt häufig zu komplexeren Produktgemischen. Ferner kann die Methylierung von Aminen auf dem Wege der reduktiven Alkylierung mittels Formaldehyd in der Regel nur in wässriger Lösung erfolgen, da Formaldehyd ausschließlich in wässriger Lösung (in der Form seines Hydrats) erhältlich ist. Dies stellt ein größeres Problem bei der reduktiven Methylierung von Aminosäuren dar, denn die in Wasser gut löslichen Aminosäuren lassen sich aus wässriger Lösung schlecht isolieren.

Die Aufgabe der vorliegenden Erfindung ist daher, ein neuartiges Verfahren für die Alkylierung von Aminen zur Verfügung zu stellen, das nicht die vorstehend erläuterten, bisher bei Verwendung von Aldehyden als Alkylierungsmittel auftretenden Nachteile aufweist.

Überraschenderweise wurde nun gefunden, dass Amine in Gegenwart von Wasserstoff und üblichen Hydrierkatalysatoren mit Orthocarbonsäureestern alkyliert werden können.

Die gestellte Aufgabe wird demgemäß gelöst durch ein Verfahren zur N-Alkylierung von Aminen durch Umsetzung eines Orthocarbonsäureesters mit einem Amin und mit Wasserstoff in Gegenwart von einem Hydrierkatalysator.

Das erfindungsgemäße Verfahren ist also ein Verfahren zur Herstellung von sekundären oder tertiären Aminen durch Umsetzung eines Orthocarbonsäureesters mit einem primären oder sekundären Amin und mit Wasserstoff in Gegenwart von einem Hydrierkatalysator. In gleicher Weise lassen sich auch primäre Amine durch Umsetzung eines Orthocarbonsäureesters mit Ammoniak und mit Wasserstoff in Gegenwart von einem Hydrierkatalysator darstellen.

Bei der Umsetzung eines Orthocarbonsäureesters mit einem Amin und mit Wasserstoff in Gegenwart von einem Hydrierkatalysator gemäß der vorliegenden Erfindung liegt das Mol-Verhältnis von Hydrierkatalysator zum eingesetzten Amin in einem Bereich von 1:10 bis 1:100.000. Der Wasserstoffpartialdruck liegt in einem Bereich von 0,1 bar bis 200 bar. Die Temperatur wird in einem Bereich von 20°C bis 200°C eingestellt.

Vorteilhaft ist es, das erfindungsgemäße Verfahren in Gegenwart von katalytischen Mengen an einer Säure durchzuführen.

Bei dem Verfahren gemäß der vorliegenden Erfindung lassen sich beispielsweise Amine mit der allgemeinen Formel (I) mit einem Orthocarbonsäureester der Formel (II) zu N-alkylierten Aminen umsetzen, wobei 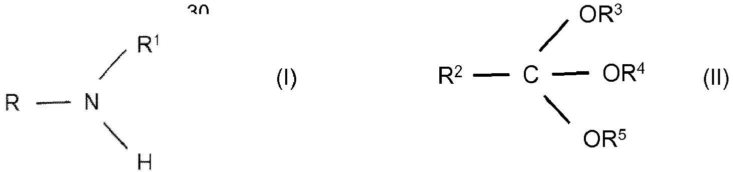
die Reste R und R¹ unabhängig voneinander ausgewählt werden aus der Gruppe bestehend aus H, linearer oder ein an einem beliebigen Kohlenstoffatom mit einem oder mehreren Substituenten versehener (C₁-C₂₄)-Alkyl-, (C₃-C₂₀)-Cycloalkyl-, (C₂-C₁₃)-Heterocycloalkyl-, (C₆-C₁₄)-Aryl-, oder (C₃-C₁₃)-Heteroaryl-Rest, mit der Maßgabe, dass R und R¹ nicht gleichzeitig H bedeuten und wobei die Reste R mit R¹ optional eine gesättigte oder einfach oder mehrfach ungesättigte (C₂-C₁₈)-Alkylen-oder (C₂-C₁₈)-Heteroalkylen-Brücke bilden können, so dass ein Ring mit insgesamt 3-20 Ringatomen entsteht,
und wobei
R² ausgewählt wird aus der Gruppe bestehend aus H, (C₁-C₂₄)-Alkyl, (C₃-C₂₀)-Cycloalkyl, (C₂-C₁₃)-Heterocycloalkyl, (C₆-C₁₄)-Aryl, (C₃-C₁₃)-Heteroaryl und
R³, R⁴ und R⁵ unabhängig voneinander ausgewählt werden aus der Gruppe bestehend aus H, (C₁-C₂₄)-Alkyl, (C₃-C₈)-Cycloalkyl oder (C₆-C₁₄)-Aryl,
wobei sowohl die Reste R² mit R³, als auch R³ mit R⁴ unabhängig voneinander eine gesättigte oder einfach oder mehrfach ungesättigte (C₂-C₁₈)-Alkylen-oder (C₂-C₁₈)-Heteroalkylen-Brücke bilden können, so dass ein Ring mit insgesamt 3-20 Ringatomen entsteht.

R² ist bevorzugt ausgewählt aus der Gruppe bestehend aus H, (C₁-C₂₄)-Alkyl, Phenyl.

R² wird besonders bevorzugt ausgewählt aus H, Methyl, Ethyl, n-Propyl, n-Butyl, iso-Butyl, n-Pentyl, n-Hexyl, Cyclohexyl, n-Heptyl, n-Octyl, Phenyl.

R³, R⁴ und R⁵ sind bevorzugt ausgewählt aus der Gruppe bestehend aus Methyl, Ethyl, n-Propyl, iso-Propyl, n-Butyl, iso-Butyl, n-Pentyl, n-Hexyl, n-Heptyl, n-Octyl, und Phenyl.

Ein Ring wird bevorzugt zwischen den Resten R² und R³ oder R³ und R⁴ gebildet, wobei der Ring bevorzugt aliphatisch ist und die Alkylengruppen Propandi-1,3-yl, Butandi-1,4-yl, Pentandi-1,5-yl, oder Hexandi-1,6-yl aufweist, so dass der Ring insgesamt 4, 5, 6, 7 oder 8 Ringatome enthält.

Die optionalen Substituenten an den Resten R und R¹ des Amins der Formel (I) sind ausgewählt aus der Gruppe bestehend aus Halogenen wie F, Cl, Br, I, und heteroatomhaltigen funktionellen Gruppen, die ein oder mehrere Atome ausgewählt aus der Gruppe bestehend aus N, O, P, S, oder Si enthalten, wobei Einfach- und Mehrfachsubstitution möglich ist. Beispiele für heteroatomhaltige funktionelle Gruppen sind Carbonyl-, Carboxyl-, Sulphonat-, Phosphonat-, Hydroxyl-, Amino-, Ammoniumgruppen wie
- OH,
- (C₁-C₈)-Alkyloxy
- COOH,
- NH({C₁-C₈}-Acyl),
- NH({C₁-C₈}-Acyloxy)
- N((C₁-C₂₀)-Alkyl)({C₁-C₈}-Acyl),
- N({C₆-C₁4}-Aryl)({C₁-C₈}-Acyl),
- N({C₆-C₁₄}-Aralkyl)({C₁-C₈}-Acyl),
- N({C₁-C₈}-Acyl)₂,
- NH₃⁺,
- NH({C₁-C₂₀}-Alkyl)₂⁺,
- NH({C₆-C₁₄}-Aryl)₂⁺,
- NH({C₆-C₁₄}-Aralkyl)₂⁺,
- NH({C₁-C₂₀}-Alkyl)({C₆-C₁₄}-Aryl)⁺,
- N({C₆-C₁₄}-Aryl)({C₁-C₂₀}-Alkyl)₂⁺,
- N({C₆-C₁₄}-Aryl)₂({C₁-C₂₀}-Alkyl)⁺,
- O-C(=O)-O-{C₁-C₂₀}-Alkyl,
- O-C(=O)-O-{C₆-C₁₄}-Aryl,
- O-C(=O)-O-{C₆-C₁₄}-Aralkyl,
- NH-C(=O)-O-{C₁-C₂₀}-Alkyl,
- NH-C(=O)-O-{C₆-C₁₄}-Aryl,
- NH-C(=O)-O-{C₆-C₁₄}-Aralkyl,
- O-C(=O)-NH-{C₁-C₂₀}-Alkyl,
- O-C(=O)-NH-{C₆-C₁₄}-Aryl,
- O-C(=O)-NH-{C₆-C₁₄}-Aralkyl,
- CN,
- SO₂-O-{C₁-C₂₀}-Alkyl,
- SO₂-O-{C₆-C₁₄}-Aryl,
- SO₂-O-{C₆-C₁₄}-Aralkyl,
- SO₂-{C₁-C₂₀}-Alkyl,
- SO₂-{C₆-C₁₄}-Aryl,
- SO₂-{C₆-C₁₄}-Aralkyl,
- SO-{C₁-C₂₀}-Alkyl,
- SO-{C₆-C₁₄}-Aryl,
- SO-{C₆-C₁₄}-Aralkyl,
- Si({C₁-C₂₀}-Alkyl)₃,
- Si({C₆-C₁₄}-Aryl)₃,
- Si({C₆-C₁₄}-Aryl)({C₁-C₂₀}-Alkyl)₂,
- Si({C₆-C₁₄}-Aryl)₂({C₁-C₂₀}-Alkyl),
- {C₁-C₂₀}-Perfluoroalkyl,
- PO(O-{C₁-C₂₀}-Alkyl)₂,
- PO(O-{C₆-C₁₄}-Aryl)₂,
- PO(O-{C₁-C₂₀}-Alkyl)(O-{C₆-C₁₄}-Aryl),
- PO({C₁-C₂₀}-Alkyl)₂,
- PO({C₆-C₁₄}-Aryl)₂,
- PO({C₁-C₂₀}-Alkyl)({C₆-C₁₄}-Aryl).

Das Amin der allgemeinen Formel (I) ist beispielsweise eine Aminosäure, beispielsweise eine der zwanzig proteinogenen Aminosäuren oder ein Aminosäurederivat, beispielsweise ein Aminosäureester, wie z.B. die Methyl- oder Ethylester von Glycin, Alanin, Valin, Leucin, Isoleucin, Phenylalanin, Asparaginsäure, Glutaminsäure, Tryptophan oder von Prolin.

Der Orthocarbonsäureester der allgemeinen Formel (II) ist beispielsweise Orthoameisensäuretrimethylester oder Orthoameisensäuretriethylester, Orthoessigsäuretrimethylester oder Orthoessigsäuretriethylester, Orthopropansäuretrimethylester oder Orthopropansäuretriethylester, Orthobutansäuretrimethylester oder Orthobutansäuretriethylester.

Orthocarconsäureester sind wohlfeil und die Herstellungsmethoden sind gut etabliert (H. Lebel, M. Grenon in Science of Synthesis, Vol. 22, 2005, pp. 669-747; G. Simchen in Methoden Org. Chem. (Houben-Weyl), 1985, E5/1, S. 3-192; R. DeWolf, Synthesis, 1974, 153-172). Sie lassen sich beispielsweise durch Alkoholyse von Nitrilen mit Alkoholen unter Säurekatalyse darstellen:

R²CN + 3 R³OH + HCl → R²-C(OR³)₃ + NH₄Cl

Orthoameisensäureester können aus Chloroform und den Natrium-Alkoholaten der entsprechenden Alkohole hergestellt werden (W. E. Kaufmann, E. E. Dreger: Ethylorthoformate. In: Organic Syntheses. 5, 1925, S. 55).

Orthocarbonsäureestern mit verschiedenen Alkoholresten (R3 ≠R4≠R5) lassen sich beispielweise durch Umesterung herstellen.

Als Hydrierkatalysator können alle dem Fachmann für diesen Zweck in Frage kommenden Hydrierkatalysatoren ausgewählt werden.

Vorzugsweise werden heterogene Hydrierkatalysatoren eingesetzt, die mindestens ein aktives Metall enthalten. Bevorzug als aktives Metall ist eines der Gruppe VII B und/oder VIII B des Periodensystems der Elemente, wobei Edelmetalle und Ni bevorzugt sind, insbesondere sind Ru, Rh, Pd, Pt, Re und Ni bevorzugt. Die Metalle können im Hydrierkatalysator entweder (a) als solche oder in Form von Metalloxiden oder (b) als Metallkomplexe vorliegen.

In Fall (a) kann das Metall oder Metalloxid entweder auf einem Träger aufgebracht oder als Partikel eingesetzt werden. Für das Trägermaterial gibt es keine Beschränkungen. Üblicherweise werden gewöhnliche Träger wie Aluminiumoxid, Siliciumdioxid, Aluminiumoxid, Eisenoxid, Magnesiumoxid, Zirkoniumdioxid, Kohlenstoff oder ähnliche dem Fachmann auf dem Gebiet der Hydrierung bekannte Träger eingesetzt. Der Gehalt an Metall oder Metalloxid auf dem Träger wird in einem Bereich von 1 Gew.-% bis 25 Gew.-% bezogen auf das Gesamtgewicht des Katalysators gewählt. Bevorzugt wird ein Gehalt von 1 bis 5 Gew.-% Metall oder Metalloxid auf dem Träger gewählt.

Beispiele für derartige Hydrierkatalysatoren sind Pt/C, Pd/C, Ru/C, Rh/C, Pd/CaCO₃, Pd/Al₂O₃, Ru/Al₂O₃, Rh/Al₂O₃, Pd/Re/C, Pt/Re/C, RuO₂.

In Fall (b) können die Metalle auch in Form von Metall-Komplexen als Hydrierkatalysatoren eingesetzt werden. Beispiele hierfür sind Metall-Komplexe der Metalle Rh, Ir oder Ru, wie z.B. der Wilkinson-Katalysator CIRh(PPh₃)₃ oder [(dppb)Rh(cod)]BF₄, [Ir(PCy₃(C₅H₅N)(cod)]PF₆, [Cl₂Ru(PPh₃)₃] und [(dppb)Ru(metallyl)₂].

Bevorzugt wird der Hydrierkatalysator ausgewählt aus der Gruppe bestehend aus Pd/C, Pd/Al₂O₃, Pd/CaCO₃, Pt/C, Ru/Al₂O₃, Ru/C, Rh/C und [(dppb)Rh(cod)]BF₄. Besonders bevorzugt wird der Hydrierkatalysator ausgewählt aus der Gruppe bestehend aus 5%Pd/C, 5%Pd/Al₂O₃, 5%Pd/CaCO₃, 5%Pt/C, 5%Ru/Al₂O₃, 5%Ru/C, 5%Rh/C und [(dppb)Rh(cod)]BF₄.

Die Menge des Hydrierkatalysators kann vom Fachmann frei gewählt werden, wobei das Mol-Verhältnis von Hydrierkatalysator zu Amin in einem Bereich von 1:10 bis 1:100.000 liegt. Weiter bevorzugt ist ein Bereich von 1:20 bis 1:10.000, besonders bevorzugt ist ein Bereich von 1:50 bis 1:2.000.

Prinzipiell ist der Fachmann frei in der Wahl des Lösungsmittels, das er in dem erfindungsgemäßen Verfahren einsetzen möchte. Aufgrund der Tatsache, dass die Ausgangsstoffe häufig in flüssiger Form vorliegen, kann insofern auf den Einsatz eines Lösungsmittels auch verzichtet werden. Wenn jedoch der Einsatz von Lösungsmitteln in dem erfindungsgemäßen Verfahren gewünscht ist, ist es vorteilhaft, solche Lösungsmittel heranzuziehen, welche die eingesetzten Komponenten der Reaktion entsprechend gut lösen und sich im Übrigen gegenüber der erfindungsgemäßen Reaktion als inert erweisen. Als solche kommen polare und unpolare Lösungsmittel, insbesondere u.a. Kohlenwasserstoffe, Chlorkohlenwasserstoffe, Ether, Ester und Alkohole in Betracht. Bevorzugt sind dabei Alkane, Halogenalkane, einwertige und mehrwertige Alkohole, cyclische und acyclische Ether, und Ester.

Bevorzugte Lösungsmittel sind solche ausgewählt aus der Gruppe bestehend aus Hexan, Heptan, Octan, Dimethylglykolether (DMGE), 1,4-Dioxan, Methyl-tert-butylether (MTBE), Tetradydrofuran (THF), Essigsäureethylester, Essigsäureisopropylester, Dibutylether, Dimethylcarbonat, Diethylcarbonat, Dipropylcarbonat, Methanol, Ethanol, Isopropanol, Butanol, Ethylenglycol, Dichlormethan, und 1,2-Dichlorethan. Besonders bevorzugt sind Methanol und Ethanol.

Bei der Reaktion wird der Wasserstoffpartialdruck der Reaktion in einem Bereich von 0,1 bis 200 bar, vorzugsweise von 0,1 bis 100 bar, und besonders bevorzugt von 0,1 bis 60 bar eingestellt.

Die Temperatur, die während der Reaktion einzustellen ist, kann vom Fachmann bestimmt werden und liegt üblicherweise in einem Bereich von 0°C bis 250°C. Sie sollte so hoch sein, dass die anvisierte Reaktion in ausreichend schneller Zeit abläuft, doch möglichst so niedrig sein, dass das Nebenproduktspektrum bei der Hydrierung so gering wie möglich gehalten werden kann. Bevorzugt wird eine Temperatur im Bereich von 20°C bis 200°C, vorzugsweise in einem Bereich von 20°C bis 150°C eingestellt. Besonders bevorzugt wird eine Temperatur im Bereich von 100°C bis 130°C eingestellt, ganz besonders bevorzugt wird eine Temperatur im Bereich von 110°C bis 130°C eingestellt.

In einer besonderen Ausführungsform des erfindungsgemäßen Verfahrens enthält der Hydrierkatalysator ein aktives Metall.

In einer weiteren besonderen Ausführungsform der Erfindung ist das aktive Metall ein Metall der Gruppe VII B und/oder VIII B des Periodensystems der Elemente.

Bei weiteren besonderen Ausführungsform der Erfindung erfolgt die Umsetzung ferner in Gegenwart einer Säure. Die Menge an Säure kann vom Fachmann frei gewählt werden. Die Säure wird jedoch bevorzugt in einem Mol-Verhältnis in einem Bereich von 0.01:100 bis 10:100 bezogen auf das Amin eingesetzt.

Prinzipiell ist der Fachmann frei in der Wahl einer geeigneten Säure. Vorzugsweise werden jedoch kostengünstige anorganische beziehungsweise organische Säuren eingesetzt. Bevorzugt sind Säuren, die ausgewählt werden aus der Gruppe bestehend aus Mineralsäuren, Carbonsäuren, Arylsulfonsäuren, Alkansulfonsäuren, Fluorsulfonsäure einzusetzen. Säuren, die ausgewählt werden aus der Gruppe bestehen aus *p*-Toluolsulfonsäure (4-MeC₆H₄SO₂OH), CF₃SO₂OH, CH₃SO₂OH, CF₃COOH, ClCH₂COOH, CH₃COOH und HCOOH sind dabei besonders bevorzugt.

Eine besondere Ausführungsform des erfindungsgemäßen Verfahrens ist, dass die Umsetzung in einem Lösungsmittel durchgeführt wird.

Das Lösungsmittel ist vorzugsweise ausgewählt aus der Gruppe bestehend aus Kohlenwasserstoffen, Chlorkohlenwasserstoffen, Ethern, Estern und Alkoholen.

Eine weitere Ausführungsform des erfindungsgemäßen Verfahrens ist, dass die Umsetzung ohne Lösungsmittel durchgeführt wird.

Es ist vorteilhaft, wasserfrei zu arbeiten. Ein weiterer Gegenstand der Erfindung ist daher ein Verfahren gemäß der vorliegenden Erfindung, wobei wasserfreies Amin und wasserfreier Orthocarbonsäureester und gegebenenfalls wasserfreies Lösungsmittel eingesetzt werden.

Bei dem erfindungsgemäßen Verfahren geht man im Allgemeinen so vor, dass man in einem Autoklaven das Amin, den Orthocarbonsäureester und den Katalysator in einem bestimmten Mol-Verhältnis mit einer geeigneten Menge Lösungsmittel vermischt. Anschließend wird der Autoklav mehrmals mit Wasserstoff gespült auf die Reaktionstemperatur erhitzt und die Mischung bei geeignetem Druck hydriert. Nach Abkühlen wird der Wasserstoffdruck abgelassen, die Reaktionsmischung wird abfiltriert und das Filtrat nach dem Fachmann bekannten Verfahren aufgearbeitet.

Bei einer bevorzugten Ausführungsform des erfindungsgemäßen Verfahrens geht man im Allgemeinen so vor, dass man in einem Autoklaven das Amin und den Orthocarbonsäureester mit einer geeigneten Menge an Lösungsmittel und Säure vermischt. Anschließend wird der Katalysator in einem bestimmten Mol-Verhältnis zugesetzt, der Autoklav wird mehrmals mit Wasserstoff gespült und die Mischung bei geeigneter Temperatur und geeignetem Druck hydriert. Nach dem Abkühlen wird der Wasserstoffdruck abgelassen, die Reaktionsmischung wird abfiltriert und das Filtrat nach einem dem Fachmann bekannten Verfahren aufgearbeitet.

Bei einer besonders bevorzugten Ausführungsform des erfindungsgemäßen Verfahrens geht man im Allgemeinen so vor, dass man in einem Autoklaven das Amin und den Orthocarbonsäureester mit einer geeigneten Menge an Säure vermischt. Anschließend wird der Katalysator in einem bestimmten Mol-Verhältnis zugesetzt, der Autoklav wird mehrmals mit Wasserstoff gespült und die Mischung bei geeigneter Temperatur und geeignetem Druck hydriert. Nach Abkühlen wird der Wasserstoffdruck abgelassen, die Reaktionsmischung wird abfiltriert und das Filtrat nach einem dem Fachmann bekannten Verfahren aufgearbeitet.

### Beispiele

Allgemeine Vorschrift für die reduktive Alkylierung von Aminen mit Orthocarbonsäureestern

Ein Autoklav wurde mit Katalysator (1 mol% bezogen auf die molare Menge an Amin) befüllt, mit Argon gespült und mit einer Lösung von Amin (0.1 mol) und Orthocarbonsäureester (0.11-0.3 mol) in 10 ml Methanol (oder Ethanol) und 0,5 ml einer 0,2 M Lösung von wasserfreier p-Toluolsulfonsäure in Methanol (oder Ethanol) aufgefüllt. Es wurde auf 120 °C aufgeheizt und 40 bar Wasserstoff aufgepresst und anschließend bei konstantem Druck gerührt, bis keine Wasserstoffaufnahme mehr zu erkennen war (0.2 - 6 h). Nach Abfiltrieren vom Katalysator wurde das Filtrat destilliert.

Die Ausbeuten können Tabelle 1 entnommen werden.

**Tabelle 1: Ausbeuten bei der reduktiven Alkylierung von Aminen mit Orthocarbonsäureestern.**

| **Beispiel Nr.** | **Orthocarbonsäureester** | **Amin** | **Produkt** | **Orthocarbonsäureester/Amin** | **Hydrierkatalysator** | **Zeit, [h]** | **Ausbeute [%]** |
|---|---|---|---|---|---|---|---|
| 1 | HC(OMe)₃ | 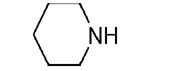 | 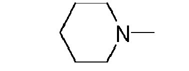 | 2.5 | 3% Pt/C | 3 | 99 |
| 2 | HC(OMe)₃ | 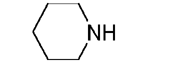 | 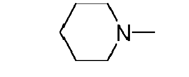 | 2.5 | 5% Ru/Al₂O₃ | 5 | 98 |
| 3 | HC(OMe)₃ | 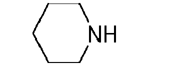 | 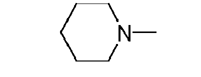 | 2.5 | [(dppb)Rh(COD)]BF₄ | 6 | 83 |
| 4 | HC(OMe)₃ | 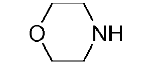 | 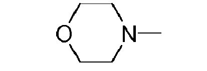 | 2.5 | 5% Pd/C | 1 | 92 |
| 5 | HC(OMe)₃ | 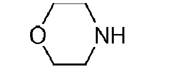 | 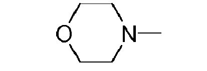 | 2.5 | 5% Pt/C | 0.2 | 97 |
| 6 | HC(OMe)₃ | 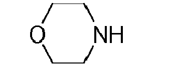 | 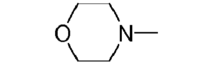 | 2.5 | 5% Ru/C | 0.3 | 96 |
| 7 | HC(OMe)₃ | 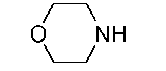 | 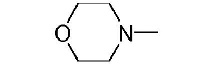 | 2.5 | 5% Rh/C | 0.3 | 98 |
| 8 | EtC(OEt)₃ | PhNH₂ | 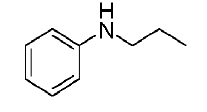 | 1.1 | 3% Pt/C | 1 | 66 |
| 9 | n-BuC(OEt)₃ | 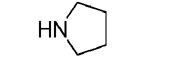 | 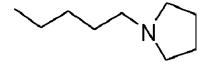 | 1.3 | 5% Pd/C | 1 | 81 |
| 10 | n-BuC(OEt)₃ | 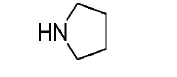 | 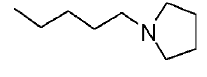 | 1.3 | 5% Pt/C | 0.2 | 95 |
| 11 | n-BuC(OEt)₃ | 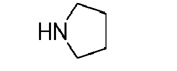 | 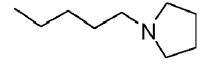 | 1.3 | 5% Ru/C | 0.2 | 90 |
| 12 | n-BuC(OEt)₃ | 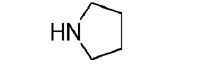 | 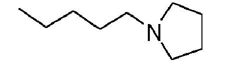 | 1.3 | 5% Rh/C | 0.2 | 86 |
| 13 | PhC(OEt)₃ | 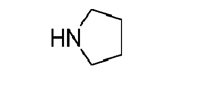 | 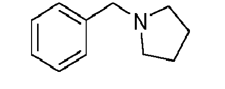 | 1.1 | 3% Pt/C | 1 | 93 |
| 14 | PhC(OEt)₃ | 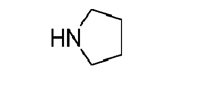 | 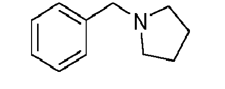 | 1.1 | 5% Rh/C | 1 | 81 |
| 15 | HC(OEt)₃ | 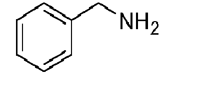 | 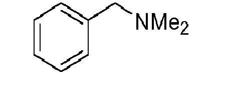 | 3 | 5% Pd/C | 1 | 99 |
| 16 | HC(OEt)₃ | 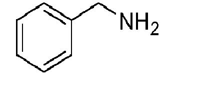 | 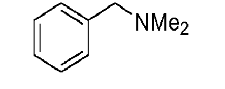 | 3 | 5% Pt/C | 0.5 | 99 |
| 17 | EtC(OMe)₃ | 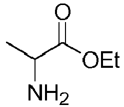 | 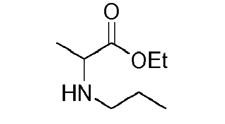 | 1.2 | 5% Pd/C | 2 | 64 |
| 18 | EtC(OMe)₃ | 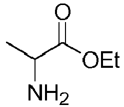 | 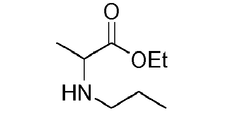 | 1.2 | 5% Pt/C | 1 | 69 |
| 19 | EtC(OMe)₃ | 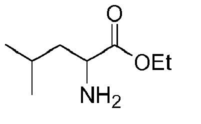 | 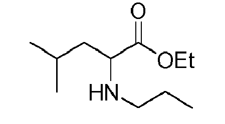 | 1.2 | 5% Pd/C | 1 | 82 |
| 20 | EtC(OMe)₃ | 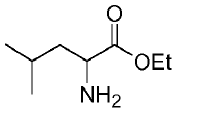 | 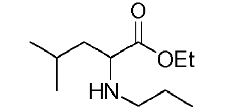 | 1.2 | 5% Pt/C | 0.1 | 75 |
| 21 | EtC(OMe)₃ | 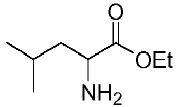 | 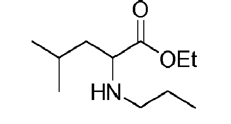 | 1.2 | 5% Ru/C | 0.5 | 71 |
| 22 | EtC(OMe)₃ | 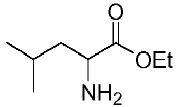 | 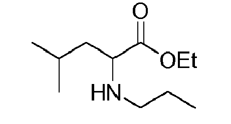 | 1.2 | 5% Rh/C | 0.1 | 85 |
| 23 | EtC(OMe)₃ | 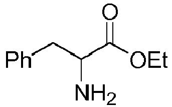 | 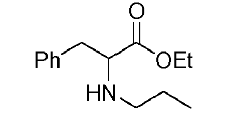 | 1.2 | 5% Pt/C | 0.1 | 83 |
| 24 | EtC(OMe)₃ | 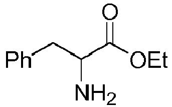 | 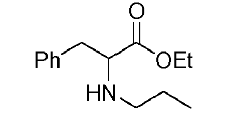 | 1.2 | 5% Ru/C | 0.2 | 84 |
| 25 | EtC(OMe)₃ | 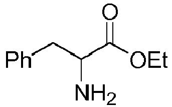 | 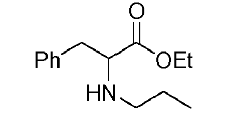 | 1.2 | 5% Rh/C | 0.2 | 90 |
| 26 | EtC(OMe)₃ | 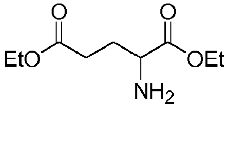 | 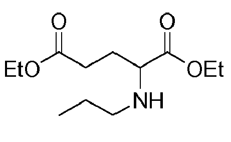 | 1.2 | 5% Ru/C | 0.5 | 93 |
| 27 | EtC(OMe)₃ | 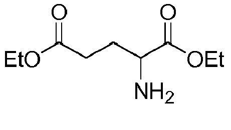 | 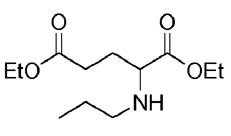 | 1.2 | 5% Rh/C | 0.1 | 95 |
| 28 | HC(OMe)₃ | 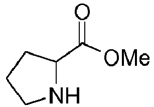 | 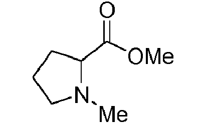 | 3 | 5% Pd/C | 0.5 | 98 |
| 29 | HC(OMe)₃ | 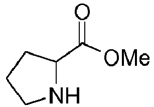 | 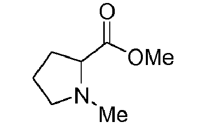 | 3 | 5% Pt/C | 0.1 | 99 |
| 30 | HC(OMe)₃ | 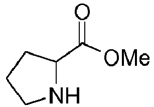 | 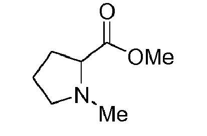 | 3 | 5% Ru/C | 0.3 | 98 |
| 31 | HC(OMe)₃ | 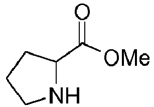 | 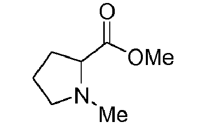 | 3 | 5% Rh/C | 0.3 | 97 |
| 32 | EtC(OMe)₃ | 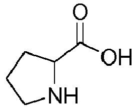 | 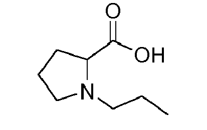 | 2 | 5% Pt/C | 0.3 | 90 |
| 33 | HC(OEt)₃ | 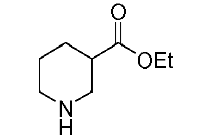 | 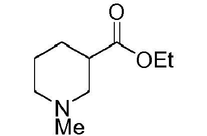 | 2 | 5% Pd/C | 0.5 | 82 |
| 34 | HC(OEt)₃ | 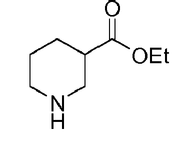 | 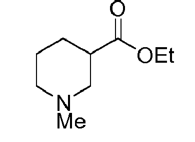 | 2 | 5% Pt/C | 0.2 | 90 |
| 35 | HC(OEt)₃ | 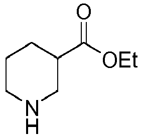 | 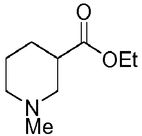 | 2 | 5% Ru/C | 0.3 | 88 |
| 36 | HC(OEt)₃ | 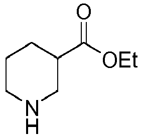 | 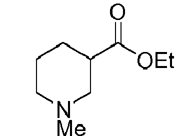 | 2 | 5% Rh/C | 0.3 | 87 |

## Patentansprüche

1. Verfahren zur N-Alkylierung von Aminen oder von Ammoniak durch Umsetzung eines Orthocarbonsäureesters mit einem Amin bzw. mit Ammoniak und mit Wasserstoff in Gegenwart von einem Hydrierkatalysator.

2. Verfahren nach Anspruch 1, wobei die Umsetzung in Gegenwart einer Säure stattfindet.

3. Verfahren nach Anspruch 2, wobei die Umsetzung bei einem Mol-Verhältnis von Säure zum eingesetzten Amin in einem Bereich von 0,01:100 bis 10:100 erfolgt.

4. Verfahren nach einem der Ansprüche 1 bis 3, wobei die Umsetzung bei einem Mol-Verhältnis von Hydrierkatalysator zum eingesetzten Amin in einem Bereich von 1:10 bis 1:100.000 erfolgt.

5. Verfahren nach einem der Ansprüche 1 bis 4, wobei die Umsetzung bei einem Wasserstoffpartialdruck in einem Bereich von 0,1 bar bis 200 bar erfolgt.

6. Verfahren nach einem der Ansprüche 1 bis 5, wobei die Umsetzung in einem Temperaturbereich von 20°C bis 200°C durchgeführt wird.

7. Verfahren nach einem der Ansprüche 1 bis 6, wobei der Hydrierkatalysator ein heterogener Hydrierkatalysator ist

8. Verfahren nach Anspruch 7, wobei der heterogene Hydrierkatalysator mindestens ein aktives Metall enthält.

9. Verfahren nach Anspruch 8, wobei das aktive Metall ein Metall der Gruppe VII B und/oder VIII B des Periodensystems der Elemente ist.

10. Verfahren nach einem der Ansprüche 1 bis 9, wobei die Umsetzung in einem Lösungsmittel stattfindet.

11. Verfahren nach Anspruch 10, wobei das Lösungsmittel ausgewählt wird aus der Gruppe bestehend aus Kohlenwasserstoffen, Chlorkohlenwasserstoffen, Ethern, Estern und Alkoholen.

12. Verfahren nach einem der Ansprüche 1 bis 11, wobei die Umsetzung ohne Lösungsmittel durchgeführt wird.
